Europäisches Patentamt

European Patent Office

Office européen des brevets

(11) Publication number: **0 141 471**
**B1**

(12) **EUROPEAN PATENT SPECIFICATION**

(45) Date of publication of patent specification: **14.03.90**

(21) Application number: **84201580.2**

(22) Date of filing: **02.11.84**

(51) Int. Cl.⁵: **C 08 K 5/12, C 08 L 27/06, A 61 L 31/00, A 61 L 33/00**

(54) Plastic compositions for biomedicaluse, and articles for biomedical use manufactured with said compositions.

(30) Priority: **07.11.83 IT 2360683**

(43) Date of publication of application:
**15.05.85 Bulletin 85/20**

(45) Publication of the grant of the patent:
**14.03.90 Bulletin 90/11**

(84) Designated Contracting States:
**AT BE CH DE FR GB LI LU NL SE**

(56) References cited:
**EP-A-0 026 912**
**BE-A- 894 719**

(73) Proprietor: **SIS-TER S.p.A.**
**Via Crema, 14**
**I-26020 Palazzo Pignano (Cremona) (IT)**

(72) Inventor: **Bertellini, Gianfranco**
**Via XX Settembre 58**
**I-22026 Maslianico(COMO) (IT)**

(74) Representative: **Dragotti, Gianfranco**
**SAIC BREVETTI s.a.s. Viale Bianca Maria, 15**
**I-20122 Milano (IT)**

Courier Press, Leamington Spa, England.

## Description

The present invention relates to a composition containing a plasticizer which is essentially non blood extractable for the manufacturing of plastic compositions, particularly polyvinyl chloride, showing an improved effect of biocompatibility and to generical articles for biomedical use manufactured with said compositions.

The recent technology aiming to the manufacturing of articles for biomedical use has shown a particular interest towards the use of plastic materials which undoubtedly contributed to the achievement of relevant and important advances in the medicine.

This is for instance the case of the emodialysis.

Among the more commonly used materials polyvinyl chloride, polyurethanes, silicone polymers and other can be cited.

In the case of polyvinyl chloride the type of plasticizer which is almost exclusively used for the preparation of plastic materials used for such a purpose is the di-2-ethylhexyl phtalate or DEHP. It has been however demonstrated that the blood, rich in lipoproteins, is very active in extracting such a compound from the polymeric materials in which it is dispersed. Suitable laboratory analysis have demonstrated the poor biocompatibility of the DEHP. The articles manufactured with these materials (particularly the bags for the blood storage) show with the time a certain decay of their flexibility properties and the blood was found at the end polluted by a compound to which recent studies attributed rather high dangerousness levels.

In order to overcome these problems, the EP—A—26912 discloses the use of the material based on polyvinyl chloride for the manufacturing of flexible bags for blood containing 30 to 50% by weight of a plasticizer consisting of tri-2-ethylhexyltrimellitate or TOTM.

This plasticizing compound is essentially obtained through esterification of trimellitic acid with 2-ethyl-hexyl alcohol namely an alcohol the main chain of which shows a branching.

More particular TOTM shows, according to the content of the said European Patent Application, a lower blood extractability and an improved biocompatibility in comparison with DEHP.

Despite the thus achieved improvement, a residual release of plasticizer to the blood remains however in the case of TOTM which is certainly an unfavourable property.

Apart from such a problem, other problems exist which are more specifically related to the use for the storage and processing of the blood, namely the behaviour at low temperatures, particularly temperatures lower than 0°C, and their elasticity, also expressed as hardness.

In fact, very often the blood is stored in bags at temperatures lower than 0°C, bags which therefor must retain the elasticity also at these temperatures and must not undergo a decay of mechanical properties with the defrosting.

According to the present invention it has now been surprisingly found that the esters of trimellitic acid with linear alcohols, either alone or in mixture, and having a number of carbon atoms of between 6 and 12, used as plasticizer, for instance for polyvinylchloride, show a release lower than that of the corresponding ester with branched alcohols, and in addition show some properties which are favourable as well, such as the increase of elasticity and a better cold flexibility, the latter properties being also obtainable with lower amounts of plasticizer in comparison with those which are normally used in the case of TOTM.

Thus the present invention, according to its main feature, consists in a plasticized composition having a low degree of release towards the blood for the preparation of plastic compositions, particularly polyvinylchloride, for biomedical use, essentially characterized in that it consists of the esters of trimellitic acid with linear alcohols, either alone or in mixture, preferably with linear alcohols having 6 to 12 carbon atoms, more preferably with 7 and 9 carbon atoms.

According to the invention there is moreover provided a composition or compound of plastic material for biomedical use characterized in that it contains 30—70% by weight, preferably 40—50%, of the plasticizer of the invention.

In the following description specific reference shall be made to the polyvinylchloride, it being the preferred example of plastic material used with the plasticizer of the invention.

The subject plasticizer may also find use, likewise and advantageously, with other suitable plastic materials, such as possibly modified cellulose polymers and copolymers of olefins with vinyl monomers.

The invention thus also contemplates the particles for biomedical use, particularly flexible articles which must come into contact with blood for more or less extended time periods, which are characterized in that they are manufactured with a compound of polyvinylchloride having the plasticizer of the invention dispersed thereinto.

Among the linear alcohols useful for the preparation of plasticizer used according to the invention there are included hexyl, heptyl, octyl, nonyl, decyl, undecyl and dodecyl alcohols.

As already mentioned, from the esterification of the trimellitic acid with these alcohols a plasticizer is obtained showing a low solubility degree into the blood and other interesting properties such as the elasticity increase and the better cold flexibility.

According to an interesting property of the invention, the amounts of plasticizer to be used are lower, with respect to those of TOTM which are conventionally used, the hardness of the final product being the same.

To this effect the effects on the increase of elasticity and of improved cold flexibility as previously cited, are to be added.

The best ratio those properties and quality of the PVC compound which can be obtained by incorporating those additives of different molecular weights has been obtained by using esters prepared from mixtures of liner $C_7$ and $C_9$ alcohols.

The articles for biomedical use manufactured with these materials, and to which the invention relates, comprise for instance hoses, bags and other generical components of the dialysis apparatus, particularly the pipes for the extra body circulation of the blood into the dialysis equipment.

Surprisingly the ester of trimellitic acid with mixtures of $C_7$ and $C_9$ alcohols has shown the following advantages with respect to the use of TOTM:

a) release to blood or like liquids lower by at least 20%;

b) improved cold flexibility; such a property is particularly important in the case of bags for the storage of frost blood, which must maintain the original flexibility also after the defrosting step;

c) an improved plasticizing power involving, the hardness being the same, a lower required concentration of the inventive plastic in PVC with respect to TOTM.

These results are quantitatively expressed in the following table in which the reported values have been obtained carrying out tests on the following materials:

Standard material (A)

| PVC (K 70) | 100 parts by weight of plastic (phr) |
| TOTM | about 30% by weight |
| Epoxidized soya oil | 3—5 parts/100 parts of plastics |
| Stabilizer based on calcium and zinc stearate | 1 part/100 parts of plastics |

Material of the invention (B)

Like (A), except that instead of TOTM about 30% by weight of a mixture of esters of trimellitic acid with linear $C_7$ and $C_9$ alcohols is used.

|  | A | B |
| --- | --- | --- |
| Hardness (shore A) | 92 | 88 |
| Behaviour at low temperature | −15°C | −24°C |
| Volatility |  | 20% respect to A |

wherein
—the hardness is measured with the value Shore A;
—the behaviour at low temperature indicates the flexibility degree at the given temperature of the plasticizer material, corresponding to the temperature at which the flexibility is essentially lost;
—the volatility indicates the weight loss of the material owing to the spontaneous evaporation of the plasticizer.

In view of the above data it seems advisable to point out the fact that the extremely advantageous behaviour of the compounds of the invention is even more surprising since nothing might indicate or even only suggest that the use of an ester obtained from linear alcohols instead of branched alcohols of the same trimellitic acid might lead to important variations of the action as plasticizer for the use in the field of the biomedical equipments.

## Claims

1. Composition of plastic material for the manufacturing of biomedical articles, characterized by containing, as the plasticizer, esters of trimellitic acid with linear alcohols containing 6 to 12 carbon atoms.

2. Composition according to claim 1, characterized in that said linear alcohols contain 7 and 9 carbon atoms.

3. Composition of plastic material containing as a dispersion 30—70% by weight of the plasticizer according to the claims 1 to 4.

4. Composition of plastic material according to claim 3, characterized by comprising plastic material containing 40—50% by weight of plasticizer and other additives.

5. Bag for the blood storage manufactured with a composition according to the claims 1 to 4.

6. Flexible pipe of a dialysis line, manufactured with the material according to the claims 1 to 4.

## Patentansprüche

1. Kunststoffmaterial für die Herstellung biomedizinischer Artikel, dadurch gekennzeichnet, dass es als Weichmacher Ester der Trimellithsäure mit linearen, 6 bis 12 Kohlenstoffatome aufweisenden Alkoholen enthält.

2. Material nach Anspruch 1, dadurch gekennzeichnet, dass die genannten linearen Alkohole 7 und 9 Kohlenstoffatome aufweisen.

3. Kunststoffmaterial, enthaltend 30 bis 70 Gew.-% des Weichmachers gemäss Ansprüchen 1 bis 4 als Dispersion.

4. Kunststoffmaterial nach Anspruch 3, dadurch gekennzeichnet, dass es 40 bis 50 Gew.-% an Weichmacher und anderen Additiven enthält.

5. Behälter zur Aufbewahrung von Blut, hergestellt mit einem Material gemäss Ansprüchen 1 bis 4.

6. Biegsamer Schlauch einer Dialyseleitung, hergestellt mit dem Material gemäss Ansprüchen 1 bis 4.

## Revendications

1. Composition de matière plastique pour la fabrication d'articles biomédicaux, caractérisée en ce qu'elle contient comme plastifiant, des esters de l'acide triméllitique avec des alcools linéaires ayant de 6 à 12 atomes de carbone.

2. Composition selon la revendication 1, caractérisée en ce que les dits alcools linéaires ont 7 et 9 atomes de carbone.

3. Composition de matière plastique, contenant en dispersion de 30 à 70% en poids de plastifiant selon les revendications 1 à 4.

4. Composition de matière plastique selon la revendication 3, caractérisée par une matière plastique contenant de 40 à 50% en poids de plastifiant et d'autres additifs.

5. Récipient pour le stockage de sang, fabriqué à partir d'une composition selon les revendications 1 à 4.

6. Tuyau flexible d'une ligne de dialyse, fabriqué à partir de la matière selon les revendications 1 à 4.